# EUROPEAN PATENT SPECIFICATION

(11) **EP 1 402 779 B1**
(45) Date of publication and mention of the grant of the patent: **02.01.2008**
(21) Application number: 02732774.1
(22) Date of filing: 24.05.2002
(51) Int. Cl.: A01N 63/02, A23B 7/155, C12N 1/20

(54) **NOVEL BIOFUNGICIDE BACTERIAL STRAIN AND THE PREPARATION METHOD AND APPLICATIONS THEREOF**
NEUER BIOFUNGIZIDER BAKTERIENSTAMM, VERFAHREN ZU SEINER HERSTELLUNG UND SEINE ANWENDUNGEN
NOUVELLE SOUCHE BACTERIENNE BIOFONGICIDE, PROCEDE DE PREPARATION DE LADITE SOUCHE ET APPLICATIONS CORRESPONDANTES

(30) Priority: 01.06.2001 ES 200101274
(43) Date of publication of application: 31.03.2004
(73) Proprietor: Universitat De Girona, 17071 Girona (ES)
(72) Inventor: MONTESINOS SEGUI, Emilio, E-17820 Banyoles (Girona) (ES); BONATERRA CARRERAS, Anna, E-17003 Girona (ES); FRANCES ORTEGA, Jesús, Manuel, E-17003 Girona (ES); BADOSA ROMANO, Esther, E-17811 Santa Pau (Girona) (ES); CABREFIGA OLAMENDI, Jordi, E-17257 Torroella de Montgri (ES)
(74) Representative: Ungria Lopez, Javier
(86) International application number: PCT/ES2002/000246
(87) International publication number: WO 2002/098233

(56) References cited:
- ES-A1- 2 149 131
- US-A- 5 783 411
- US-A- 5 919 446
- NUNES C. ET AL.: 'Biological control of postharvest pear diseases using a bacterium Pantoea agglomerans CPA-2' INTERNATIONAL JOURNAL OF FOOD MICROBIOLOGY vol. 70, 2001, pages 53 - 61, XP002961445
- BRYK H. ET AL.: 'Antagonistic effect of Erwinia herbicola on in vitro spore germination and germ tube elongation of Botrytis cinerea and Penicillium expansum' BIOCONTROL vol. 43, 1998, pages 97 - 106, XP000995992
- SHOLBERG P.L. ET AL.: 'Biological control of postharvest pathogens of apple with antagonistic microorganism' PHYTOPATHOLOGY vol. 84, no. 10, 1994, page 1152, XP000986240

## Description

### TECHNICAL FIELD OF THE INVENTION

The present invention is to be placed within the agri-food sector and in particular in the phytosanitary protection of crops.

More specifically, the present invention provides a new strain of the bacterial species Erwinia herbicola (Pantoea agglomerans) with biofungicidal properties which has important applications in the protection of plants in general and, in particular, of crops, especially collected fruits, against attack by phytopathogenic microorganisms.

### PRIOR ART

The demands of national and international market leads to an adaptation of agricultural supply and production and a demand that is more and more independent of the harvest season, forcing the productive sectors to lengthen the shelf-life of the fruits and vegetables and to maintain their organoleptic quality during cold storage preservation, commercialisation and home storage.

Thus, for example, apples and pears are preserved in large industrial warehouses frequently for periods of 6 to 10 months in cold storage preservation under controlled atmosphere conditions. Although it is difficult to make a precise estimate, annual global losses due to diseases, including rot and physiological disorders, in post-harvest have been calculated at between 5 and 30% of production, the great majority of which are caused by fungi. The main fungal rots are caused by Penicillium expansum and Botrytis cinerea in apples and pears, Penicillium italicum and P. digitatum in citrus fruits and Monilia spp. nd Rhizopus stolonifer in peaches, apricots, plums and other stone fruits (Snowdon AL, CRC Press. 1990; Jones and Aldwinckle, APS Press, 1990; Montesinos E. et al. Mundi Prensa, 2000). The majority of infections are produced as a consequence of mechanical injuries during the handling of the plant products and subsequent fungal attack, above all during the stages of introduction, closure and set up of the store room environment (which can take various days, even weeks), and during packaging and commercialisation.

Controlling these diseases in the fruit and vegetable warehouses is carried out by means of refrigeration (-1 to 1°C), control of the gases in the atmosphere of the store rooms (CO₂, O₂), minimising mechanical handling, inoculum reduction and disinfection, and applying synthetic fungicides or calcium salts to the fruit. However, the use of synthetic chemical fungicides presents a series of problems, among which the most noteworthy are those deriving from disposal or the elimination of the treatment liquids, the presence of residues in the fruit and the appearance of resistance in the fungi causing the rot (Eckert JW and Ogawa JM. Annual Review of Phytopathology 26:433-469, 1988; Gullino M and Kuijpers CAN. Annual Review of Phytopathology 32:559-579, 1994). Furthermore, the progressive implantation of Integrated Fruit Production (IFP) quality label has led to a selective and rational use of synthetic fungicides during the production phase in the field, and as a consequence, depending on each case, to an elimination or minimisation of post-harvest chemical treatments.

Biological control of the fungi causing post-harvest rot is currently an alternative to the synthetic fungicides (Wilson and Wisniewski, Annual Review of Phytopathology 27:425-441, 1989; Cook RJ, Annual Review of Phytopathology 31:53-80, 1993; Gullino ML, Informatore Fitopatologico 9:5-13, 1994). The process of biocontrol or bioprotection of fruit with antagonistic microorganisms is based mainly on competitive exclusion or effective colonisation of the points of entry of the pathogen (lenticels or wounds), direct antibiosis (including enzymes) against the pathogen and inducing resistance in the fruit which generally means the healing of the mechanical wounds and blocking the infection of the pathogen. The use of microorganisms to inoculate the fruit in pre-harvest or post-harvest is the object of an increasing number of applications which in some cases have led to invention patents which include antagonistic microorganisms such as bacterial strains of Pseudomonas cepacia, Pseudomonas syringae and Bacillus subtilis; yeasts such as Candida oleophila, Candida sake, Pichia quilliermondii, Cryptococcus laurenti and Kloeckera apiculata; and fungi such as Acremonium breve and various species of Trichoderma, which have shown various degrees of efficiency against Penicillium expansum, Penicillium digitatum, Penicillium italicum, Botrytis cinerea, Rhizopus, Monilia, Mucor, Alternaria which produce rot in pome, stone or citrus fruits (Chalutz E and Wilson CL, Plant Disease 74:134-137, 1990; Chand-Goyal T and Spotts RA, Postharvest Biology and Technology 7:51-64, 1996; Droby S et al., Phytopathology 87:310-315, 1997; Huang Y et al., Postharvest Biology and Technology 3:293-304, 1993; Janisiewicz WJ and Marchi A, Plant Disease 76:555-560, 1992; McLaughlin RJ et al., Phytopathology 80:456-461, 1990; McLaughlin RJ et al., Plant Disease 76:470-473, 1992; Mercier J and Wilson CL, Postharvest Biology and Technology 6:9-15, 1995; Pratella GC and Mari M, Postharvest Biology and Technology 3:49-56, 1993; Pusey PL et al., Plant Disease 72:622-626, 1988; Roberts RG, Phytopathology 80:526-530, 1990; Sanderson PG and Spotts RA, Phytopathology 85:103-110, 1995; Smilanick JL and Denis-Arrue R, Plant Disease 76:481-485, 1992; Teixid6 N et al., Phytopathology 88:960-964, 1998).

However, in spite of the advances that have been achieved in this sector, the technology of biofungicide production continues to raise various technical problems, the solutions to which would be highly desirable.

One of the main problems that biofungicides have is the fact that they are, in general, less effective than synthetic fungicides, for which reason higher concentrations of biofungicidal microorganisms are required in order to achieve comparable efficiency. Very often the effective dose for treatment is excessively large for it to be produced on a commercial scale, bearing in mind the maximum cell concentration that can be obtained by fermentation, which makes the system unviable due to the difficulty in achieving a profitable industrial process of obtaining the biomass. In most of the cases described in the literature on the use of bacteria, the doses required to get good efficiency are, in general, very high. As an example we can cite the results published concerning Bacillus subtilis B-3 in peaches with optimal doses of 1-5 x 10⁸ cfu/ml (Pusey PI et al., Plant Disease 72:622-626, 1988), Pseudomonas syringae L59-66 in pears with optimal doses of 5 x 10⁸ cfu/ml (Janisiewicz WJ and Marchi A, Plant Disease 76:555-560, 1992) and Pseudomonas cepacia ID2131 in citrus fruits with optimal doses of 1 x 10⁹ cfu/ml (Huang Y et al., Postharvest Biology and Technology 5:129-137, 1995).

On the other hand, among the currently existing agents for biocontrol of post-harvest rot, the yeasts are without doubt the most widely-used, but, since they are unicellular fungi, they are sensitive to many of the fungicides used in agriculture, and, in general, have limited compatibility with chemical treatments, especially anti-scald agents, salts or fungicides and waxes, that are used before and after harvest.

A third inconvenience with some biofungicides, especially those that are quite efficient, even comparable to synthetic fungicides, and above all among those whose active components are bacteria, is that they produce, in vitro, appreciable quantities of antibiotics or antimicrobial secondary metabolites whose potential production in the plant product may limit their acceptability in the eyes of the health authorities or the consumers. This has been demonstrated in strains of Bacillus subtilis (McKeen CD et al., Phytopathology 76:136-139, 1986), Pseudomonas fluorescens (Gutterson N, Critical Reviews in Biotechnology 10:69-91, 1990) and Pseudomonas syringae (Bender SC et al., Microbiological and Molecular Biology Reviews 63:266-292, 1999). Also in Erwinia herbicola the percentage of strains producing antibiotics is relatively high (Ishimaru et al., Phytopathology 78:746-750, 1988; Wodzinsky RS and Paulin JP, Journal of Applied Bacteriology 76:603-607, 1994).

To give an example, we need to cite the patents for the strains of Erwinia herbicola whose application consists precisely in the production of antibiotics such as herbicolins (Winkelmann G, UPS 4.338.302) or carbapenems (Kempf JA and Wilson KE, USP 4.247.640; Sykes RB and Wells JJ, UPS 4.405.716).

There are even cases, such as some strains of Pseudomonas fluorescens (Von Graevenitz, Annual Review of Microbiology 31:447-471, 1977) or Pseudomonas cepacia (Rozee KR, Diagnostic Microbiology and Infectious Disease 20:181-186, 1994), that may be plant or opportunistic human pathogens.

Another problem that exists with some biofungicides of a bacterial nature, and which supposes a serious limitation for its practical use in certain cases, is the low survival rate of the cells, above all on the surface of the fruit and vegetables, including in the injuries, due to the high osmotic potential of the juice. This is the consequence of a low water availability. Furthermore, in general, a major loss of viability occurs during the preparation or preservation procedures which result in water elimination (deep freezing, dehydration, lyophilisation).

Finally, in terms of the potential for industrial production of biofungicides, in many cases difficulties exist in production in fermentors in liquid culture at high biomass and in achieving repetitiveness in quality, as well as problems in formulation and preservation of the preparation. Another aspect of the practical applications is the lack of consistency in pilot trials and tests under real conditions, due to the fact that they are strongly affected by factors related to the variety or the host plant species to be protected, and the pathogen which causes the rot.

Up to now, the majority of patents that involve strains of Erwinia herbicola have applications in frost damage control, in various crops (Arny DC and Lindow SE, USP 4045910 and USP 4161084; Lindow SE, USP 4432160) or for the control of fire blight, a disease that affects certain rosaceous plant species caused by Erwinia amylovora (Pusey PL, USP 5919446). There does exist a patent in which a description is given of the CECT4920 strain of Erwinia herbicola for biological control of post-harvest fungal rot (Vinas et al., PCT/ES00/00101) but the description of the strain and the claims made for it only include the phenotypic and metabolic characteristics by means of the API 20E and BIOLOG GN tests whose results do not always allow the differences between strains to be established nor do they include specific genotypic markers (for example RFLPs) that would allow it; they do not exclude its possible pathogenicity in plants through proper tests; they do not indicate if the strain produces antibiotics; they do not provide toxicological or pathogenic studies in animals and nor do they establish the procedures for its cultivation on an industrial scale or formulation of it for its preservation and commercialisation.

The applicants have channelled their investigative efforts into the search for a strain capable of overcoming the difficulties described in the foregoing paragraphs; these efforts have culminated in the discovery of a new strain of the bacterial species Erwinia herbicola (Pantoea agglomerans), which constitutes the basis of the present invention, and which will be described in detail in the following sections of this descriptive report.

### DETAILED DESCRIPTION OF THE INVENTION

The present invention, as indicated in its announcement, refers to a new strain of the bacterial species Erwinia herbicola (Pantoea agglomerans), with fungicidal activity which protects plants from attack by phytopathogenic microorganisms. Furthermore, the present invention refers to compositions that the said strain contains, or of extracts containing the same, or of compositions that contain compounds derived from the same, all of which are capable of protecting plants and, especially, the fruits in the postharvest phase from attack by the aforementioned microorganisms. The present invention also includes any mutant derived from the wild strain that has essentially the same characteristics. Likewise, the invention refers to the procedures for obtaining the said strain or its derivatives on a laboratory, semi-industrial or industrial scale, and the applications of the same in the protection of plants and, especially, of the fruits.

Throughout this report, we will describe the characteristics of the pure culture of the bacteria Erwinia herbicola, strain EPS125 which possesses phenotypic and genotypic molecular markers which distinguish it from other isolates of the same species, and which does not present toxicity in either plants or animals, nor does it produce known antibiotics. Furthermore, it will be seen how the strain is highly effective as an agent of biological control in the prevention of fungal rot, caused mainly by Penicillium expansum and other fungi in fruit and vegetables during cold storage and commercialisation.

In this report, we shall also describe the procedures for mass cultivation on a semi-industrial scale in a fermentor, treatment and formulation for optimising efficiency, preservation methods of the formulated product and the methodology of its application, which permits it to be produced and utilised on an industrial scale. Likewise, we shall illustrate the procedures for the preparation of biofungicides which use the said strain EPS125, as well as the results of evaluations of its activity in semi-industrial conditions of application.

All of the above shall illustrate how the present invention resolves the various technical problems that the sector faces and which have been described in the preceding section of this descriptive report.

The strain EPS125 (this is the identification reference assigned by the applicant) was deposited in the Spanish Type Culture Collection in accordance with the conditions stipulated in the Budapest Treaty, with the following identification: Pantoea agglomerans CECT 5392 (for this reason, from now on, either of the two references used to designate the strain of this invention may be used indistinguishably). The said strain corresponds to the species Erwinia herbicola (Enterobacter agglomerans, Pantoea agglomerans, Pantoea herbicola) in accordance with the *Bergey's Manual of Systematic Bacteriology* and others (Dye DW, New Zealand Journal of Agricultural Research 21: 153-177, 1978; Ewing WH and Fife MA, International Journal of Systematic Bacteriology 22 : 4-11, 1972; Gavini F et al., International Journal of Systematic Bacteriology 39: 337-345, 1989).

Characterisation by means of microbiological and biochemical tests, as well as by means of the API 20E system, is as follows:
- Produces colonies with orange pigmentation in Luria Bertani (LB) or nutrient (NA) agar, grows in Miller-Scroth (MS) medium producing characteristic orange colonies.
- At the microscopic level, the cultures are made up of mobile, Gram negative bacilli.
- Its metabolism is characterised by oxidation/fermentation (+/+),
   ONPG (+), ADH (-), LDC (-), ODC (-), citrate (-), hydrogen sulphide (-), urease (-), tryptophan deaminase (+), indole (-), Voges-Proskauer (+), gelatinase (+), oxidase (-), glucose (+), mannose (+), inositol (-), sorbitol (-), rhamnose (+), sucrose (+), melibiose (+), starch (+), arabinose (+), nitrate reduction (+).

The strain presents a characteristic electrophoretic pattern of macrofragments of digestion of genomic DNA with restriction endonuclease enzymes, Xba I and Swa I, which differentiate it from other isolates of the same species. This pattern consists of 12 bands of 306, 268, 255, 246, 233, 178, 153, 146, 141, 120, 87 and 77 Kbases with the Xba I enzyme, and 13 bands of 312, 304, 290, 235, 188, 168, 162, 150, 141, 122, 121, 111 and 86 Kbases with the enzyme Swa I. The pattern was obtained following the procedure used by Rainey et al. (Microbiology 140: 2315-2331, 1994): the pure cultures of the strain grown in an LB broth were centrifuged and were included in blocks of agarose, and then lysed in an EDTA-laurylsarcosine-proteinase K buffer for 48 hours at 56°C, and finally, there followed the digestion of the DNA by the restriction enzyme at 37°C for 24 hours. The resulting fragments of the DNA digestion ("inserts"), were transferred to agarose gels and the fragments were separated by means of the CHEF procedure of electrophoresis in a pulsating field using incremental pulses of 5 to 25 seconds for 21 hours at 200 V and 120 mA.

The pattern of the Restriction Fragment Length Polymorphisms (RFLPs) of the DNA of the strain EPS125 is highly specific given that it differentiates it from the other strains of Erwinia herbicola, as is shown in the following Table.

**Table 1. Number and size of the resulting fragments of the digestion of DNA by means of restriction enzyme Xba I in the interval from 50 to 310 Kb, in various strains of Erwinia herbicola in comparison with the strain EPS125. The size of the bands can vary by more or less 5Kb.**

| Strain | Number of frag ments | Size of the fragments (Kilobases) |
|---|---|---|
| EPS125 | 12 | 77, 87, 120, 141, 146, 153, 178, 233, 246, 255, 268, 306 |
| EPS130 | 12 | 89, 108, 121, 146, 154, 177, 244, 260, 268, 282, 290, 298 |
| EPS156 | 10 | 110, 134, 147, 188; 203, 220, 230, 262, 270, 287 |
| EPS283 | 9 | 63, 76, 94, 109, 157, 175, 239, 278, 290 |
| EPS307 | 7 | 129, 164, 173, 232, 240, 272, 300 |
| EPS325 | 6 | 138, 147, 174, 226, 259, 293 |
| EPS405 | 12 | 62, 71, 103, 111, 144, 154, 166, 172, 197, 217, 234, 274 |
| EPS435 | 11 | 54, 78, 94, 103, 124, 142, 215, 228, 252, 265, 287 |
| EPS453 | 8 | 59, 78, 99, 119, 193, 220, 247, 264 |
| EPS475 | 11 | 56, 80, 128, 176, 221, 228, 245, 251, 267, 279, 287 |
| EPS547 | 11 | 60, 81, 116, 127, 163, 178, 201, 238, 258, 271, 290 |

The pattern of RFLPs is stable in all the culture conditions that were studied and even months after being inoculated into apples and pears in cold storage conditions.

Furthermore, the strain presents a resistance marker to rifampicin (60 microgrammes/ml) which allows it to be counterselected in selective culture media supplemented with this antibiotic inhibiting the growth of other microorganisms present in the sample that are not resistant to rifampicin whose frequency is generally less than 10⁻⁹ and is useful for carrying out studies of traceability together with the PFGE pattern.

The mutant was obtained by means of selecting a spontaneously resistant colony obtained from seeding a confluent culture in LB agar supplemented with rifampicin by means of the gradient method, and therefore without any genetic manipulation of the original strain.

The strain EPS125 thus characterised allows a favourable resolution to be given to most of the problems and inconveniences that currently exist in the sector.

Thus, for example, compared with the high effective doses of the biofungicides used up to now, the median effective dose of the strain EPS125 in bioassays on the inhibition of infection by P. expansum in apples and pears is somewhere between 10⁶ and 10⁷ cfu/ml.

These tests were carried out at pathogen doses with levels of rot incidence approaching 100%; the fruits, in which they had made wounds, were mature and treated with various concentrations of the strain by means of immersion, were incubated for 6 to 12 hours at 20°C, were inoculated with the pathogen by pulverised spray at a dose of 5 x 10⁴ conidia/ml, they were then incubated at 20°C for 5 to 7 days and the percentage of infected wounds was determined. Table 2, which follows, shows the results of these tests.

**Table 2. Mean incidence of infected wounds per fruit depending on the concentration of the biocontrol agent. The experimental design consisted of 3 repetitions of 6 fruits with 6 to 9 injuries per fruit.**

| Dose (cfu/ml) | EPS125* |
|---|---|
| 0 | 95 a |
| 10⁴ | 90 a |
| 10⁵ | 78 ab |
| 10⁶ | 40 b |
| 10⁷ | 21 c |
| 10⁸ | 10 d |
| 10⁹ | 5 de |

| | |
|---|---|
| "*" Different letters in the same column indicate significant differences according to Duncan's mean separation test. | |

The strain EPS125 as well as showing high efficiency when applied preventively before the inoculation of the pathogen P. expansum, is also effective when applied simultaneously or 6 hours after the pathogen. This is a unique aspect which distinguishes the strain of the present invention from other agents of biocontrol and which endows it with a certain curative power. Table 3, which follows, summarises this aspect of the experiment.

**Table 3. Effect of the timing of the inoculation of the pathogen with respect to the application of the biofungicidal treatment (strain EPS125), in the incidence of rot in pears, of the variety Passe Crassane, preserved for 15 days at 5°C.**

| | Concentration of inoculum of Penicillium expansum (conidia/ml) | |
|---|---|---|
| Time (hours) (*) | 10³ | 10⁴ |
| Control | 100 | 100 |
| - 6 (beforehand) | 22 | 33 |
| 0 (simultaneously) | 22 | 33 |
| + 12 (afterwards) | 11 | 22 |
| + 24 (afterwards) | 11 | 22 |

| | | |
|---|---|---|
| (*) time at which the pathogen was inoculated with respect to the biofungicide, the experimental design consisted of 3 repetitions of 3 pears with nine wounds in each pear. | | |

Compared to the inconvenient sensitivity of the currently existing biocontrol agents for post-harvest rot, to many of the chemical fungicides normally used in agriculture, or to their limited compatibility with the conventionally used chemical treatments in pre- and post-harvest, the strain EPS125 is tolerant of the following fungicides: Folpet, Imazalil, Myclobutanil, Thiabendazole, Thiram, Benomyl, Captan, Procymidone, Tebuconazole, Diphenylamine and Methyl-thiophanate. It is sensitive to derivatives of copper, kasugamycin, streptomycin and guazatine, although these four products are not a problem given that they are not authorised for post-harvest use. This natural tolerance was demonstrated in tests on the growth-inhibiting activity by means of the technique of incorporation in agar at the dose used in commercial application for various phytosanitary products. The tolerance towards the fungicides is without doubt due to the bacterial nature of the strain. The strain EPS125 is also compatible with the application of salts such as calcium chloride and sodium bicarbonate at the concentrations that are frequently used in post-harvest.

Compared to the inconvenience of some biofungicides which produce, in vitro, appreciable quantities of antibiotics or anti-microbial secondary metabolites, one of the peculiarities of strain EPS125 is that it produces no antibiosis "in vitro" when tested in a dual culture against Erwinia amylovora, Pseudomonas syringae, Penicillium expansum or Stemphylium vesicarium, in different culture media such as Glucose- Asparagine (GA), King B (KB), Luria-Bertani (LB) or Potato-Dextrose (PD), whether supplemented or not with Fe.

Furthermore, compared to the potentially pathogenic nature of certain current biofungicides to plants or humans, the strain EPS125 is neither pathogenic nor toxic in the plants or animals in which it has been inoculated or administered. When it was infiltrated into tobacco leaves using Klement's hypersensitivity test no HR reaction was produced. (Lelliot RA and Stead DE, Blackwell Scientific Publications, 1987). Inoculation of it in various host plants did not produce infection nor symptoms of disease from which it can be deduced that it is, in general, not phytopathogenic, as is shown by the existence of a wide literature base in which it has been demonstrated that the Erwinia herbicola species is not pathogenic in plants (Dye DW, New Zealand Journal of Agricultural Research 21:153-177, 1978; Ewing WH and Fife MA, International Journal of Systematic Bacteriology, 22:4-11, 1972; Gavini F et al., International Journal of Systematic Bacteriology 39:337-345, 1989). Only two strains have been described of Erwinia herbicola named pv. gypsophilae and pv. betae that are pathogenic in Gypsophila paniculata and beet (Cooksey DA, Plant Disease 70:464-468, 1986), and they present characteristics that differentiate them from the rest of the strains of the species among which there stands out the hypersensitivity reaction in tobacco.

It is also not active in ice nucleation, as is seen by the absence of freezing of cell suspensions above-9°C, according to the Lindow procedure (Lelliot RA and Stead DE, Blackwell Scientific Publications, 1987). In tests with laboratory animals it did not produce primary irritation in the eyes nor eye injuries in rabbits up until the seventh day of treatment (OECD 405, Directives 92/69/EEC Appendix B-B.5, and 93/21/EEC). It also did not produce primary skin irritation nor skin injuries in rabbits (OECD 404, Directives 92/69/EEC Appendix B-B4, and 93/21/EEC). The studies of acute toxicity in mice (OECD 401, Directives 92/69/EEC Appendix B-B.1) indicate that the LD₅₀, when administered orally by means of stomach tube in mice, is greater than 10⁸ cfu/Kg, with none of the mice dying and all developing normal body weight.

Another problem of the currently existing biofungicides of a bacterial nature is the low survival rate of their cells on the surfaces of the fruit and vegetables that they are meant to treat. In contrast, the strain EPS125 inoculated in pears and apples at doses of 10⁶, 10⁷ or 10⁸ reaches population levels on the skin surface of between 10⁴ and 10⁵ cfu/cm², but in the case of wounds occurring, these populations increase in these injuries up to 10⁷⁻10⁸ cfu/cm², after 12-24 hours at 15°C and after 5-10 days at 4°C with the consequent protection against infections by P. expansum and other fungi. These levels are maintained for various weeks and months depending on the conditions of preservation. These results indicate that one of the main mechanisms that occurs in the activity of the strain EPS125 as a biofungicide is the colonisation and competitive exclusion of possible pathogens in the wounds. In Table 4 which follows, the results of the experiments carried out with respect to this are summarised.

**Table 4. Levels of the severity of rot (diameter of the lesions) and survival of the strain EPS125 in Golden Delicious apples and Passe Crassane pears in different conditions of preservation and concentrations of the applications. The viable count was carried out in LB agar supplemented with rifampicin (50 µg/ml).**

| | | | Population level log₁₀ (cfu/cm²) | |
|---|---|---|---|---|
| Test | Treatment * | Mean diameter of the rot (mm) | Wounds | Surface |
| Golden | Control | 46.3 | -- | -- |
| Delicious apples | 10⁶ cfu/ml | 18.9 14.2 | 7.22 7.32 | 4.10 4.20 |
| 15 days at 15°C (injury- | 10⁷ cfu/ml | 13.6 | 7.76 | 5.35 |
| treatment-inoculation) | 10⁸ cfu/ml | | | |
| Golden | Control | 48.9 | -- | -- |
| Delicious apples | 10⁸ cfu/ml | 2.8 | 7.78 | 5.23 |
| 12 days at 4°C | | | | |
| 14 days at 15°C | | | | |
| (injury-treatment-inoculation) | | | | |
| Passe Crassane | Control | 47.0 | -- | -- |
| pears 15 days at 4°C | 10⁶ cfu/ml | 31.3 25.7 | 6.69 5.51 | 3.92 4.40 |
| 10 days at 15°C (treatment- | 10⁷ cfu/ml | 25.0 | 8.10 | 4.82 |
| injury-inoculation) | 10⁸ cfu/ml | | | |

| | | | | |
|---|---|---|---|---|
| * The inoculation with *P. expansum* was carried out at a concentration of 10⁴ conidia/ml and was applied 6-12 hours after treatment with the biofungicide. The experimental design consisted of each treatment in 3 fruits with 12 injuries per fruit. | | | | |

One of the main obstacles for the development of production technology for biofungicides lies in the need to test thousands of isolates of micro-organisms until a few potentially useful ones are found, given their low frequency in nature. The strain EPS125 which is the object of the present invention was obtained using a procedure of selective enrichment "inplanta". The selective enrichment method consists of cultivating the crude extracts of plants in the plant material itself (for example, fruits) and then inoculating the pathogen. Only those extracts which after being enriched in the plant material inhibit the infection by the pathogen are processed in order to obtain pure cultures of the representative component microorganisms of the sample. This selective enrichment procedure avoids the inconvenience of the classical methods which consist of directly isolating the pure culture of the representative microorganisms of the sample and testing a posteriori their activity as an agent of biocontrol, and results in a ten-fold improvement in performance in terms of obtaining the biocontrol agent compared to the classical method. In the process of isolating the strain EPS125, the samples of plants were homogenised in buffered peptone water and the extract containing the microorganisms was concentrated by centrifugation. The concentrate, resuspended in the buffer, was applied to discs of apple to which P. expansum was subsequently inoculated. The strain was obtained by means of the usual procedures of isolation and obtaining the pure culture, using the extracts from samples that proved effective in the tests of inhibition of apple infection. Beginning from 200 samples of plant material from 30 plant species, various climatic zones, including organs such as roots, leaves, flowers and fruits, a total of 1000 isolates were produced. Specifically, the strain EPS125 came from the fruit of a pear tree Pyrus communis of the Doyenne du Comice cultivar.

The strain EPS125 can be stored in the laboratory by means of techniques used for the preservation of bacteria in general. Preservation in the short term is achieved by means of reseeding in LB agar and maintenance at 2-4°C; in the medium term (1-3 years) by means of deep freezing of suspensions at -80°C in 20% glycerol; and in the long term (various years), by means of lyophilisation in suspensions in skimmed milk. The culture medium affects slightly the activity of the strain as a biofungicide, with the most suitable being the Luria-Bertani medium and a semi-complex medium called MSD composed of: glucose, 10g/l; yeast extract, 4 g/l; K₂HPO₄, 4 g/1; KH₂PO₄, 3.67 g/l; Na₂HPO₄, 5.73 g/l; (NH₄)₂ SO₄, 1.2 g/l; NH₄ Cl, 0.2 g/l; CaCl₂·2H₂O, 0.04 g/l; FeSO₄·7H₂O, 0.04 g/l; MgSO₄·7H₂O, 0.0024 g/l; incubated at a temperature of 25-27°C at pH = 7.0.

In the MSD medium, after 8-10 hours, the culture reaches a stationary phase and a cell concentration is achieved of 3-8 x 10⁹ cfu/ml corresponding to 3-5 g fresh weight/l, and yield of 6 x 10⁸ cfu/g of glucose or 0.4 g fresh weight/g of glucose. In conditions of osmotic adaptation the stationary phase is delayed by up to 2-4 hours, with respect to cultivation in the LB broth.

For scaling-up and mass production in a 75 litre fermentor (50 litre of operational volume) Biostat-UD (B. Braun Biotech) the following procedure was used: A pre-inoculum culture of 1-2 litres in MSD medium was prepared and, once the stationary phase was achieved after 8-10 hours, this was used to inoculate the bioreactor (4% v/v). The correct operating conditions are 25-28°C, pH 7.0, an agitation speed of 250-400 rpm, and an air flow of 30-60 lpm. After 4-5 hours a solution of 200-400 g of glucose is added and fermentation continues for a further 8-12 hours. Then, the mixture undergoes continuous centrifugation at 10,000 rpm and 20-30 1/h and the cells are collected aseptically using a continuous centrifuge CSA-1 (Westfalia-Separator) maintaining the operating conditions of the bioreactor during the 2-3 hours that the separation process lasts. At the end of this stage, the biomass is concentrated by a factor of 10-12, resuspended in a phosphate buffer, 0.2M (pH 7.0), in order to obtain between 4-5 litres of 3 -4 x 10¹⁰ cfu/ml (40-50 g fresh weight/1). The total harvest of the fermentation of 50 1 corresponds to approximately 10¹¹ cfu and 200-250 g fresh weight. The potential of a fermentation of this kind, using a commercial dose of around 1-5 x 10⁷ cfu/ml, is for between 2,000 and 10,000 litres of liquid treatment. By means of the manipulation of the fermentation using the osmoadaptive system described, spontaneous microcolonies are formed with the subsequent improvement in survival during processes of dehydration and separation from the biomass.

The survival capacity and efficiency of the strain EPS125 can be significantly increased by means of an osmoadaptive procedure. This procedure consists of inducing the cells, during their cultivation, to accumulate certain osmoprotective substances in the presence of high concentrations of a salt, although the most efficient osmoadaptive agent depends on each type of microorganism (Miller KJ and Wood JM, Annual Review of Microbiology 50:101-136, 1996). This method can be used to increase the tolerance of microorganisms to situations of low water availability (activity), which is a condition that occurs during the colonisation of the wounds of the fruits with juices that have high osmotic potential, or on the surface of the plant or fruit when relative humidity is low, as well as during the preparation of formulations by means of dehydration, lyophilisation or deep freezing. The strain EPS125 becomes osmoadaptive when betaine (3-5 mM) and sodium chloride (0.5-0.7 M) are added to the culture medium. In these conditions, a singularity of the strain is that it forms microaggregates (microcolonies) of 10-50 micrometres in size absorbed in a matrix of exomucopolysaccharide of an unknown nature which keeps them in suspension, a fact which favours their formulation (see subsequent sections). Table 5 summarises the results of the experiments in relation to the osmoadaptive conditions.

**Table 5. Mean absorbance in the stationary phase of liquid cultures of the strain EPS125 and a strain of Pseudomonas fluorescens EPS288 in minimal medium depending on the osmoadaptive conditions.**

| Treatment | Strain | |
|---|---|---|
| | EPS125 | EPS288 |
| Control | 0.795 | 0.780 |
| Betaine +NaCl 0.7 M | 0.760 | 0.410 |
| Choline +NaCl 0.7 M | 0.380 | 0.580 |
| Trehalose +NaCl 0.7 M | 0.300 | 0.160 |
| NaCl 0.7 M | 0.310 | 0.130 |

This osmoadaptation has clear influence in the significant increase in the efficiency level of biocontrol of blue mould rot, as was shown in comparative tests with the biofungicide produced without osmoadaptation, as shown in Table 6.

**Table 6. Effect of osmoadaptation on the efficiency of the strain EPS125 in the control of mean diameter (mm) of blue mould rot in apples and pears, in relation to the osmoadaptation during the preparation of the inoculant. The fruit was disinfected, injuries were made with a spike, treatments of biofungicides were applied and 24 hours later they were inoculated with P. expansum (10⁴ conidia/ml). The material was incubated at 15°C for 11 days.**

| Test * | Untreated Control | Dose cfu/ml | | | |
|---|---|---|---|---|---|
| | | 10⁷ | | 10⁸ | |
| | | Control | Osmoadapted | Control | Osmoadapted |
| . Apple | 45.0 | 21.9a^{y} | 13.1 b | 13.4 a | 8.6 b |
| . Pear | 40.0 | 17.8 a | 8.5 b | 11.4 a | 9.1 b |
| . Pear | 48.1 | 10.3 a | 9.1 a | 14.4 a | 6.4 b |

| | | | | | |
|---|---|---|---|---|---|
| * The test consisted of two doses of the strain EPS125 and an untreated control. Each treatment with the strain EPS125 consisted of an osmoadapted inoculant and a non-osmoadapted control. The experimental design consisted of 6 fruits with 9-12 injuries per fruit for each treatment. ^{y} The different letters for each pair of control/osmoadapted comparisons for each dose of biofungicide indicate the existence of significant differences with the Duncan mean separation test. | | | | | |

The concentrated material produced in fermentation on a semi-industrial scale and with osmoadaptation, in the way described above, remains active at 1-4°C for various days. If it is deep frozen it can maintain its fungicide activity and its cell viability for months.

To commercialise the biofungicidal product described in this present invention, the material thus preserved can be lyophilised or dehydrated by atomisation, which gives it a useful life of various months.

When the lyophilised or atomised product is to be used, it is reconstituted with non-chlorinated water to prepare the treatment liquid which is applied to the fruit by spraying or immersion before its preservation in cold storage.

The combination of the strain EPS125 with a synthetic fungicide produces a synergy that leads to a greater efficiency of the resulting mixture than that of the two components separately. This system of combination of a fungicidal product at low or standard concentration is especially useful as a strategy against resistance developing in the rot-causing pathogen. Furthermore, another advantage of the strain EPS125 is that it tolerates well the remains of chemical products in the application equipment and these do not therefore require an exhaustive cleaning in order to be used for the biofungicide, as is the case with other biocontrol agents.

The tests that have been carried out with the strain described in this present invention demonstrate that its efficiency is comparable with conventional fungicidal treatments, in a wide range of orchards and storage conditions.

According with what has been said above, the main application of the strain EPS125 or any of its extracts or active derivatives, or mutants which have essentially the same phenotypic, biochemical or molecular markers, is in the manufacture of biofungicidal preparations for the protection of fruits, before and after harvest, from infections arising from phytopathogenic fungi, as well as the facilities, equipment and containers that are destined to come into contact with the fruit.

The said biofungicidal preparations may, in addition, include a conventional chemical fungicide compatible with the said strain or its derivatives, as well as an agriculturally acceptable carrier or vehicle.

The biofungicidal preparation is especially suitable for the treatment of infections caused by phytopathogenic fungi, especially those belonging to the Penicillium genus. Likewise, the said preparation can be applied to any type of fruit, but it is especially ideal for pome fruits.

### MEANS OF USE OF THE INVENTION

This invention is additionally illustrated by means of the following Examples, which are not to be considered as the limits of its range, defined exclusively in the Claim Form attached.

### EXAMPLE 1

### a) Isolation of the strain EPS125

5g of material composed principally of the skin of fruit from the pear tree Pyrus communis of the Doyenne du Comice cultivar was homogenised in 20 ml of sterile buffered peptone water (peptone 0.5 g/l in phosphate buffer, 0.1M at pH 7.0) using a blade homogeniser (Stomacher system). The extract containing the microorganisms present in the fruit were left to settle and the supernatant was centrifuged at 5000xg for 15 minutes. The sediment obtained with the microorganisms was resuspended in 5 ml of phosphate buffer 0.1 M pH =7.0 (henceforth we shall refer to this as the extract). Using Golden Delicious apples, whose surfaces had been disinfected by immersion for 1 minute in a solution of sodium hypochlorite (1% w/v) and washed various times in order to eliminate the remaining hypochlorite, cylindrical discs were prepared measuring 1 cm in diameter and 1 cm deep in which wound, of approximately 2 mm diameter and the same depth, was made in the skin. Then 9 discs were taken and 10 microlitres of the extract was applied to 6 of them leaving 3 discs untreated. The material was incubated for 12 hours at 25°C in a humid chamber (80-90% relative humidity). Afterwards, in the wound made to each disc, 5 microlitres were applied of a solution of conidia of P. expansum at a concentration of 4 x 10⁴ conidia/ml, and the discs were once again incubated at 25°C for 5 days in the humid chamber. In the case of the discs treated with the extract in which no rot was formed, these were then processed to extract the component microorganisms by means of the procedure described previously. The extract obtained underwent the usual techniques used in microbiology to isolate pure cultures in solid media of the different microorganisms it is composed of. In the specific sample where the strain EPS125 was isolated, 99 % of the bacterial colonies that grew corresponded in morphology and colonial nature to this strain. After successive stages of subculture using individual colonies, its purity was verified by means of obtaining colonial homogeneity. Finally, its activity in the control of blue mould rot was tested by means of the test for inhibition of infection by P. expansum in discs of Golden Delicious apples described previously.

### b) Maintenance, scaling-up and fermentation of the strain EPS125

The maintenance and preservation conditions for the strain EPS125, obtained in line with stage a) above, were as follows:
Short term: reseeding in LB agar, and kept at 2-4°C;
Medium term (1-3 years) by means of deep freezing in suspensions of glycerol at 20% (w/v), at minus 80°C;
Long term (various years), by means of lyophilisation in suspensions in skimmed milk in the same conditions as described in section d) below.

For scaling-up and mass production in a 75 litre fermentor (50 operational litres) Biostat-UD (B. Braun Biotech), the following procedure is used: A pre-inoculum culture of 1.5 litres in MSD medium is prepared and, once the stationary phase is achieved after 9 hours, this is used to inoculate the bioreactor (4% v/v). The correct operating conditions are 27°C, pH 7.0, an agitation speed of 300 rpm, and an air flow of 45 lpm. After 4½ hours a solution of 300 g of glucose is added and fermentation continues for up to 10 hours. Then, the mixture undergoes continuous centrifuging at 10,000 rpm and 25 1/h and the cells are collected aseptically using a continuous centrifuge CSA-1 (Westfalia-Separator) maintaining the operating conditions of the bioreactor during the 2½ hours that the separation process lasts. At the end of this stage, the biomass is concentrated by a factor of 11, resuspended in a phosphate buffer, 0.2M (pH 7.0), in order to obtain between 4-5 litres of 3 - 5 x 10¹⁰ cfu/ml (45 g fresh weight/1). The total harvest of the fermentation of 50 1 corresponds to approximately 10¹¹ cfu and 225 g fresh weight.

### c) Improving the efficiency of the strain EPS125 by means of osmoadaptation

The strain EPS125 is osmoadapted by treatment with betaine (4 mM) and sodium chloride (0.7 M) of the culture medium of the process described in section b) above.

In order to carry out the osmoadaptive process on the scale of a semiindustrial fermentor, the same procedure is used as in normal conditions, excepting that the MSD culture medium is complemented with sodium chloride (0.7 M) and betaine (4 mM) (which shall henceforth be called modified MSD). With the aim of avoiding an excessive phase of latency of the culture in the fermentor at the semiindustrial scale, the preinoculum culture is prepared in modified MSD.

### d) Formulation and preservation of the biofungicidal preparation for commercial use

The concentrated material produced in fermentation on a semi-industrial scale (50 1), which contains 3-5 x 10¹⁰ cfu / ml (45 g fresh weight/l) obtained in the procedure described in the previous section is maintained active at 1-4°C for various days. It can be deep frozen at -70/80°C, adding glycerol at a final concentration of 15% v/v and maintained at -20°C. In these conditions the fungicidal activity and cell viability can be maintained for some months.

It can also be prepared by means of lyophilisation. To do this, sterilised skimmed milk or dextran is added at a final concentration of 5-10% or inositol at 5%. The conditions of lyophilisation in a preparative lyophiliser are as follows: The samples are frozen on trays at -45°C. A primary drying under a vacuum is carried out by temperature decrease of +0.5-1°C/min to a temperature of - 10°C (about 3 hours) maintaining the sample in a vacuum for 20 hours until the temperature of the sample reaches 0°C. Then, secondary drying takes place and is carried out at 0.3-0.5°C/min until 15°C is reached (about 5 hours). In these conditions a fall of 0.69 log₁₀ cfu occurs, but the viability remains stable for months at room temperature in a dry environment.

Another preparation method consists of spray drying. The conditions of the spraying process are as follows: The concentrate is prepared by adding xanthan gum, dried skimmed milk or gelatinised starch at 5% in order to get a minimum final dry extract of 10%. The correct parameters in a semiindustrial atomiser are a flow of 0.7 l/hour, temperature of incoming air 125°C and temperature of outgoing air 43°C. In these conditions there is a fall of 1 log₁₀ cfu, but viability remains stable for months at room temperature in a dry environment.

Both preparations, obtained by either lyophilisation or spraying allow the biofungicidal product to be commercialised with a useful life of several months.

In order to use and prepare the treatment liquid, the lyophilised or atomised concentrate is diluted by 0.5 g fresh weight/l with non-chlorinated water and is applied to the fruit by spraying or immersion before being preserved in cool storage.

### EXAMPLE 2

In this example, the association of the biofungicidal strain of this invention, EPS125, with a conventional synthetic fungicide is described.

In Table 7, which follows, the tests carried out on pears of the Conference variety and various commercial fungicides are described.

**Table 7. Incidence of rot ten days after inoculation of a strain of P. expansum resistant to thiabendazole, in tests on pears of the Conference variety kept for 10 days at 15°C- 95% RH and subjected to different treatments.**

| Treatment * | incidence (%) |
|---|---|
| Control | 100 |
| Thiabendazole (1.2 g m.a./l) | 96 |
| EPS288 (10⁸ cfu/ml) | 61 |
| Imazalil (0.037 g m.a./1) | 13 |
| Folpet 1.6 (g m.a./1) | 6 |
| EPS125 (10⁸ cfu/ml) | 4 |
| Imazalil 0.37 (commercial | 0 |
| dose g m.a./l) | 0 |
| EPS125 (10⁸ cfu/ml) + Imazali | |
| 0.037g m.a./l) | |

| | |
|---|---|
| * The experimental design consisted of 8 fruits with 6-9 injuries per fruit | |

### EXAMPLE 3

In this example some semi-commercial tests on the post-harvest efficiency of the biofungicide which is the object of this invention shall be described.

The pilot trials carried out in cold storage warehouses during the years 1995, 1996 and 1997 in Catalonia, La Rioja and Navarra using apples and pears from various commercial orchards show that the efficiency of the strain EPS125 at a dose of 1 x 10⁸ cfu/ml is comparable to that of conventional fungicide treatments, under a wide range of environmental, farming and preservation conditions.

Table 8, which follows, shows the conditions in which these tests were carried out and the results obtained.

**Table 8. Mean incidence of blue mould rot in pears and apples from various commercial orchards in storage tests* in fruit and vegetable centres. The preservation conditions were at room temperature for 3 weeks (RT, 20°C) or in cold storage in controlled atmosphere (CA) for 4-6 months (3% oxygen, 3% carbon dioxide, 1°C).**

| Test | Year | Preser vation conditions | Number of farms | Untreated control | Fungicide | Biofungicide |
|---|---|---|---|---|---|---|
| Golden | 1995 | CA | 11 | 7.9 a | 0.5 b | 0.5 b |
| Delici | 1995 | RT | 11 | 23.9 a | 3.0 b | 4.1 b |
| ous | 1996 | RT | 11 | 24.7 a | 2.7 b | 1.9 b |
| apple | 1997 | CA | 11 | 6.9 a | 4.2 b | 0.3 c |
| Confe | 1996 | RT | 6 | 21.9 a | 0.0 b | 0.1 b |
| rence | 1996 | CA | 6 | 19.8 a | 0.9 b | 2.0 b |
| pear | 1997 | CA | 9 | 16.3 a | 0.1 c | 1.9 b |

| | | | | | | |
|---|---|---|---|---|---|---|
| * The experimental design consisted of three treatments per orchard. Between 6-12 hours after each treatment a 2-4 x 10⁴ conidia/ml solution of *P*. *expansum* was applied. Each treatment consisted of 3-5 repetitions, 6 fruits per repetition and 6-9 injuries per fruit. ^{y} The fruits were treated by immersion in water (control fruit), with a fungicidal mixture consisting of imazalil (0.37 g m.a./l) and folpet (1.2 g m.a./l), or with a biofungicidal solution of 1 x 10⁸ cfu/ml of the strain EPS125. | | | | | | |

## Claims

1. New biofungicidal bacterial strain, **characterised in that** it is the strain EPS125 of the bacterial species Erwinia herbicola (Pantoea agglomerans), deposited in the Spanish Type Culture Collection with the access number CECT 5392, or an extract or active derivative of the same, or a mutation of the wild strain, all of which have essentially the same phenotypic, biochemical or molecular markers, distinctively specific to the same.

2. Biofungicidal bacterial strain, according to claim 1, **characterised by** the fact that it has the following microbiological and biochemical properties:
- Produces colonies with yellow pigmentation in Luria Bertani (LB) or nutrient agar (NA);
- grows in Miller-Schroth (MS) medium producing characteristic orange colonies;
- the cultures, at the microscopic level, are made up of mobile Gram-negative bacilli;
- metabolism **characterised by** oxidation /fermentation(+/+), ONPG (+), ADH (-), LDC (-), ODC (-), citrate (-), hydrogen sulphide (-), urease (-), tryptophan deaminase (+), indole (-), Voges-Proskauer (+), gelatinase (+), oxidase (-), glucose (+), mannose (+), inositol (-), sorbitol (-), rhamnose (+), sucrose (+), melibiose (+), starch (+), arabinose (+), nitrate reduction (+).
- an electrophoretic pattern of macrofragments of digestion of genomic DNA via restriction endonuclease enzymes Xba I and Swa I, consisting of 12 bands of 306, 268, 255, 246, 233, 178, 153, 146, 141, 120, 87 and 77 Kbases with Xba I, and 13 bands of 312, 304, 290, 235, 188, 168, 162, 150, 141, 122, 121, 111 and 86 Kbases with Swa I;
- possesses a resistance marker to rifampicin.

3. Bacterial biofungicidal strain, according to claim 1, **characterised by** the fact that it possesses a median effective dose of 10⁶-10⁷ cfu/ml against Penicillium expansum .

4. Bacterial biofungicidal strain, according to claim 3, **characterised by** the fact that it is also effective when applied simultaneously and up to 6 hours after the said pathogen Penicillium expansum.

5. Bacterial biofungicidal strain, according to claim 1, **characterised by** the fact that it is compatible with synthetic fungicides selected from among Folpet, Imazalil, Myclobutanil, Thiabendazole, Thiram, Benomyl, Captan, Procymidone, Tebuconazole, Diphenylamine and Methyl-thiophanate.

6. Bacterial biofungicidal strain, according to claim 1, **characterised by** the fact that it is compatible with the application of salts at the concentrations used normally in post-harvest.

7. Bacterial biofungicidal strain, according to claim 6, **characterised by** the fact that the said salts are calcium chloride and sodium bicarbonate.

8. Bacterial biofungicidal strain, according to claim 1, **characterised by** the fact that it produces no antibiosis "in vitro" when tested in dual cultures against Erwinia amylovora, Pseudomonas syringae, Penicillium expansum or Stemphylium vesicarium, in different culture mediums such as glucose-asparagine, King B, Luria-Bertani or potato-dextrose, whether supplemented or not with Fe.

9. Bacterial biofungicidal strain, according to claim 1, **characterised by** the fact that it is neither pathogenic nor toxic for plants, animals or humans.

10. Bacterial biofungicidal strain, according to claim 1, **characterised by** the fact that it has a high cell survival rate on the surfaces of fruit and vegetables treated with it.

11. Bacterial biofungicidal strain, according to claim 10, **characterised by** the fact that the said survival rate is of the order of 10⁴-10⁵ cfu/cm² inoculated in pears and apples at doses between 10⁶-10⁸ cfu/cm², and whose population increases up to 10⁷-10⁸ cfu/cm² after 12-24 hours at 15°C and after 5-10 days at 4°C when wounds are produced in the skin surface of these fruits.

12. Procedure for obtaining the new bacterial strain CECT 5392 defined in accordance with the above claims 1 to 11, **characterised in that** it consists of the homogenisation of the fruit from the pear tree Pyrus communis of the variety Doyenne du Comice in buffered peptone water; obtaining an extract containing the microorganisms from the corresponding homogenate followed by concentration of it by centrifugation; applying the said concentrated extract previously diluted in a buffer to discs of apple that are later inoculated with P. expansum, and finally, the isolation and preparation of a pure culture from those extracts that show themselves to be effective at inhibiting infection.

13. Procedure, according to claim 12, **characterised in that** the strain thus obtained can be maintained and preserved in the short term by means of reseeding in LB agar, at 2-4°C.

14. Procedure, according to claim 12, **characterised in that** the strain thus obtained can be maintained and preserved in the medium term, that is, 1 to 3 years, by means of deep freezing at -80°C of solutions of the strain in glycerol at 20% (w/v).

15. Procedure, according to claim 12, **characterised in that** the strain thus obtained can be maintained and preserved in the long term (several years) by means of lyophilisation of solutions of the strain in skimmed milk.

16. Procedure, according to claim 12, **characterised in that** the strain thus obtained can be obtained on a large scale by means of mass production in a fermentor using the following operations:
a) a pre-inoculum culture is prepared in MSD medium until the stationary phase is achieved, which is then used to inoculate the bioreactor operating at 25-28°C, pH 7.0, 250-400 rpm and an air flow of 30-60 1pm. After 4-5 hours, a solution of 200-400 g of glucose is added and fermentation continues for up to 8-12 hours;
b) continuous centrifugation at 10,000 rpm and 20-30 1/h and the cells are collected aseptically, maintaining the operating conditions of the bioreactor for 2-3 hours;
c) the biomass from the reactor is concentrated for 2-3 hours and resuspended in a phosphate buffer, to obtain the strain on a large scale.

17. Procedure according to any of the claims 12 to 16, **characterised by** the fact that the strain CECT 5392 can be improved in terms of its fungicidal efficiency by means of an additional stage of osmoadaptation which is carried out by treating it with betaine and sodium chloride.

18. Use of the strain CECT 5392 defined by any one of the above claims 1 to 11, in the manufacture of biofungicidal preparations for commercial use that includes the said strain or an extract or active derivative of the same, or a mutant of the same, which has essentially the same specific molecular or phenotypic markers.

19. Use according to claim 18, **characterised in that** the said biofungicidal preparations incorporate, in addition to the strain, a conventional chemical fungicide.

20. Use according to claim 19, **characterised in that** the said conventional chemical fungicide is selected from a group made up of Folpet, Imazalil, Myclobutanil, Thiabendazole, Thiram, Benomyl, Captan, Procymidone, Tebuconazole, Diphenylamine and Methyl-thiophanate

21. Use according to any of the claims 18 to 20, **characterised in that** the said biofungicidal preparations are found to be mixed, formulated or diluted with an agriculturally acceptable compound in order to improve its adherence, wettability, to provide nutrients and/or to improve the chances of survival of the strain.

22. Use according to any of the claims 18 to 21, **characterised in that** it is directed towards controlling fungal infections produced by phytopathogenic fungi.

23. Use according to claim 22, **characterised in that** the said phytopathogenic fungus belongs to the genus Penicillium.

24. Use according to any of the claims 18 to 23, **characterised in that** it is directed towards the protection of fruit against fungal infections.

25. Use according to claim 24, **characterised in that** the said fruit is protected in the pre-harvest state, applying the said biofungicidal preparations to the trees in the fields where they are cultivated.

26. Use according to claim 24, **characterised in that** the said fruit is protected in the post-harvest state during the period it is stored and preserved.

27. Use according to claims 24 to 26, **characterised in that** the said fruit is pome fruit.

28. Use according to any of the claims 18 to 27, **characterised in that** said strain is applied to the installations, equipment or containers destined to come into contact with the said fruit.

## Patentansprüche

1. Neuer biofungizider Bakterienstamm, **dadurch gekennzeichnet, dass** er der Stamm EPS125 der Bakterienspezies Erwinia herbicola (Pantoea agglomerans), hinterlegt bei der Spanish Type Culture Collection unter der hinterlegungsnummer CECT 5392, ist, oder ein Extrakt oder aktives Derivat von diesem oder eine Mutation des Wildstamms, die alle im Wesentlichen die gleichen phänotypischen, biochemischen oder molekularen Marker aufweisen, die spezifisch sind für diesen.

2. Biofungizider Bakterienstamm nach Anspruch 1, **dadurch gekennzeichnet, dass** er die folgenden mikrobiologischen und biochemischen Eigenschaften aufweist:
- erzeugt Kolonien mit gelber Pigmentierung in Luria Bertani (LB) oder Nähragar (NA);
- wächst in Miller-Schroth-Medium (MS-Medium), wobei charakteristische orange Kolonien erzeugt werden;
- die Kulturen setzen sich auf der mikroskopischen Ebene aus mobilen Gramnegativen Bazillen zusammen;
- Metabolismus, **gekennzeichnet durch** Oxidation/Fermentation (+/+), ONPG (+), ADH (-), LDC (-), ODC (-). Citrat (-), Schwefelwasserstoff (-), Urease (-), Tryptophandesaminase (+), Indol (-), Voges-Proskauer(+), Gelatinase (+), Oxidase (-), Glucose (+), Mannose (+), Inositol (-), Sorbitol(-); Rhamnose (+), Sucrose (+), Melibiose (+), Stärke (+), Arabinose (+), Nitratreduktion (+);
- ein elektrophoretisches Muster von Makrofragmenten des Verdaus von genomischer DNA **durch** Restriktionsendonukleaseenzyme XbaI und Swa I, bestehend aus 12 Banden von 306, 268. 255, 246, 233, 178, 153, 146, 141, 120, 87 und 77 KBasen mit Xba I und 13 Banden von 312,304, 290, 235, 188, 168, 182, 150, 141, 122, 121, 111 und 86 KBasen mit Swa I;
- besitzt einen Resistenzmarker für Rifampicin.

3. Biofungizider Bakterienstamm nach Anspruch 1. **dadurch gekennzeichnet, dass** er eine mittlere effektive Dosis von 10⁶-10⁷ KBE/ml gegen Penicillium expansum besitzt.

4. Biofungizider Bakterienstamm nach Anspruch 3, **dadurch gekennzeichnet, dass** er auch wirksam ist, wenn er gleichzeitig und bis zu 6 Stunden nach dem Pathogen Penicillium expansum angewandt wird.

5. Biofungizider Bakterienstamm nach Anspruch 1 **dadurch gekennzeichnet, dass** er mit synthetischen Fungiziden, ausgewählt aus Folpet, Imazalil, Myclobutanil, Thiabendazol, Thiram, Benomyl, Captan, Procymidon, Tebuconazol, Diphenylamin und Methyl-thiophanat kompatibel ist.

6. Biofungizider Bakterienstamm nach Anspruch 1. **dadurch gekennzeichnet, dass** er mit der Anwendung von Salzen in den normalerweise nach der Ernte verwendeten Konzentrationen kompatibel ist.

7. Biofungizider Bakterienstamm nach Anspruch 6, **dadurch gekennzeichnet, dass** die Salze Calciumchlorid und Natriumhydrogencarbonat sind.

8. Biofungizider Bakterienstamm nach Anspruch 1. **dadurch gekennzeichnet, dass** er keine Antibiose "in vitro" hervorruft, wenn er in dualen Kulturen gegen Erwinia amylovora, Pseudomonas syringae, Penicillium expansum oder Stemphylium vesicarium in verschiedenen Kulturmedien wie Glucose-Asparagin, King B, Luria-Bertani oder Kartoffel-Dextrose, mit, Fe ergänzt oder nicht ergänzt, getestet wird.

9. Biofungizider Bakterienstamm nach Anspruch 1, **dadurch gekennzeichnet, dass** er weder pathogen noch toxisch für Pflanzen, Tiere oder Menschen ist.

10. Biofungizider Bakterienstamm nach Anspruch 1, **dadurch gekennzeichnet, dass** er eine hohe Zellüberlebensrate auf der Oberfläche von damit behandelten Früchten und Gemüsepflanzen aufweist.

11. Biofungizider Bakterienstamm nach Anspruch 10, **dadurch gekennzeichnet, dass** beim Einimpfen in Birnen und Äpfel in Dosen zwischen 10⁶-10⁸ KBE/cm² die Überlebensrate in der Größenordnung von 10⁴-10⁵ KBE/cm² liegt, und seine Population sich auf bis zu 10⁷-10⁸ KBE/cm² nach 12-24 Stunden bei 15 °C und nach 5-10 Tagen bei 4 °C erhöht, wenn Wunden auf der Oberfläche der Schale dieser Früchte erzeugt werden.

12. Verfahren zum Erhalten des neuen Bakterienstamms CECT 5392, der gemäß den vorstehenden Ansprüchen 1 bis 11 definiert ist, **dadurch gekennzeichnet, dass** es die Homogenisierung der Frucht von dem Birnbaum Pyrus communis der Varietät Doyenne du Comice in gepuffertem Peptonwasser; das Erhalten eines Extrakts, der die Mikroorganismen aus dem entsprechenden Homogenat enthält, gefolgt von einer Konzentration desselben durch Zentrifugation; das Aufbringen des zuvor in einem Puffer verdünnten konzentrierten Extrakts auf Apfelscheiben, welche später mit P. expansum beimpft werden, und schließlich die Isolierung und Herstellung einer Reinkultur aus denjenigen Extrakten umfasst, welche sich ais wirksam zum Verhindern einer Infektion erweisen.

13. Verfahren nach Anspruch 12, **dadurch gekennzeichnet, dass** der so erhaltene Stamm kurzfristig durch Wiederaussäen in LB-Agar bei 2-4 °C aufrechterhalten und konserviert werden kann.

14. Verfahren nach Anspruch 12, **dadurch gekennzeichnet, dass** der so erhaltene Stamm mittelfristig, d. h. 1 bis 3 Jahre, durch Tiefgefrieren bei -80 °C von Lösungen des Stamms in Glycerin mit einer Konzentration von 20 % (Gew./Vol.) aufrechterhalten und konserviert werden kann.

15. Verfahren nach Anspruch 12, **dadurch gekennzeichnet, dass** der so erhaltene Stamm langfristig (mehrere Jahre) durch Lyophilisierung von Lösungen des Stamms in Magermilch aufrechterhalten und konserviert werden kann.

16. Verfahren nach Anspruch 12, **dadurch gekennzeichnet, dass** der so erhaltene Stamm in großem Maßstab durch Massenproduktion in einem Fermenter erhalten werden kann unter Verwendung der folgenden Arbeitsgänge:
a) eine Impfmaterial-Vorkuftur wird in MSD-Medium hergestellt, bis die stationäre Phase erreicht ist, welche dann verwendet wird, um den Bioreaktor zu beimpfen, der bei 25-28 °C, pH 7,0, 250-400 U/min und einem Luftstrom von 30-60 l/min betrieben wird, wobei nach 4-5 Stunden eine Lösung von 200-400 g Glucose zugegeben wird und die Fermentation bis zu 8-12 Stunden fortgesetzt wird;
b) kontinuierliche Zentrifugation bei 10000 U/min und 20-30 l/h und die Zellen werden aseptisch gesammelt, wobei die Betriebsbedingungen des Bioreaktors 2-3 Stunden beibehalten werden;
c) die Biomasse aus dem Reaktor wird 2-3 Stunden konzentriert und in einem Phosphatpuffer resuspendiert, um den Stamm in großem Maßstab zu erhalten.

17. Verfahren nach einem der Ansprüche 12 bis 16, **dadurch gekennzeichnet, dass** der Stamm CECT 5392 hinsichtlich seiner fungiziden Wirksamkeit durch einen zusätzlichen Osmoadaptionsschritt verbessert werden kann, weicher durch Behandeln des Stamms mit Betain und Natriumchlorid ausgeführt wird.

18. Verwendung des Stamms CECT 5392, der durch einen der vorstehenden Ansprüche 1 bis 11 definiert ist, bei der Herstellung von biofungiziden Zubereitungen zur kommerziellen Verwendung, welche den Stamm oder einen Extrakt oder ein aktives Derivat von diesem oder eine Mutante von diesem einschließen, welche im Wesentlichen die gleichen spezifischen molekularen oder phänotypischen Marker aufweisen.

19. Verwendung nach Anspruch 18, **dadurch gekennzeichnet, dass** die biofungiziden Zubereitungen zusätzlich zu dem Stamm ein herkommliches chemisches Fungizid enthalten.

20. Verwendung nach Anspruch 19, **dadurch gekennzeichnet, dass** das herkömmliche chemische Fungizid ausgewählt ist aus einer Gruppe, bestehend aus Folpet, Imazalil, Myolobutanil, Thiabendazol, Thiram, Benomyl, Captan, Procymidon, Tebuconazol, Diphenylamin und Methyl-thiophanat.

21. Verwendung nach einem der Ansprüche 18 bis 20, **dadurch gekennzeichnet, dass** die biofungiziden Zubereitungen mit einer landwirtschaftlich annehmbaren Verbindung vermischt, formuliert oder verdünnt sind, um ihre Anhaftung, Benetzbarkeit zu verbessern, um Nährstoffe bereitzustellen und/oder um die Überlebenschancen des Stamms zu verbessern.

22. Verwendung nach einem der Ansprüche 18 bis 21, **dadurch gekennzeichnet, dass** sie auf das Bekämpfen von Pilzinfektionen gerichtet ist, die durch phytopathogene Pilze hervorgerufen werden.

23. Verwendung nach Anspruch 22, **dadurch gekennzeichnet, dass** der phytopathogene Pilz zu der Gattung Penicillium, gehört.

24. Verwendung nach einem der Ansprüche 18 bis 23, **dadurch gekennzeichnet, dass** sie auf den Schutz von Früchten vor Pilzinfektionen gerichtet ist.

25. Verwendung nach Anspruch 24, **dadurch gekennzeichnet, dass** die Frucht in dem Zustand vor der Ernte geschützt wird, wobei die biofungiziden Zubereitungen auf die Bäume in den Feldern aufgebracht werden, wo sie kultiviert werden.

26. Verwendung nach Anspruch 24, **dadurch gekennzeichnet, dass** die Frucht in dem Zustand nach der Ernte während des Zeitraums, in dem sie gelagert und konserviert wird, geschützt wird.

27. Verwendung nach den Ansprüchen 24 bis 26, **dadurch gekennzeichnet, dass** die Frucht eine Kernfrucht ist.

28. Verwendung nach einem der Ansprüche 18 bis 27, **dadurch gekennzeichnet, dass** der Stamm auf die Anlagen, Ausrüstung oder Behälter aufgebracht wird, die dazu bestimmt sind, mit der Frucht in Berührung zu kommen.

## Revendications

1. Nouvelle souche bactérienne biofongicide, **caractérisée en ce qu'**il s'agit de la souche EPS125 de l'espèce bactérienne *Erwinia herbicola (Pantoea agglomerans),* déposée auprès du Spanish Type Culture Collection sous le numéro d'accès CECT 5392, ou un extrait ou un dérivé actif de celle-ci, ou une mutation de la souche sauvage, tous ayant essentiellement les mêmes marqueurs phénotypiques, biochimiques ou moléculaires, distinctivement spécifiques de celle-ci.

2. Souche bactérienne biofongicide selon la revendication 1, **caractérisée par le fait qu'**elle possède les propriétés microbiologiques et biochimiques suivantes :
- elle produit des colonies avec une pigmentation jaune dans un milieu Luria Bertani (LB) ou une gélose nutritive (NA) ;
- elle se développe dans un milieu Miller-Schroth (MS) produisant des colonies orange caractéristiques ;
- les cultures, au niveau microscopique, sont constituées par des bacilles Gram négatif mobiles ;
- un métabolisme **caractérisé par** : oxydation/fermentation (+/+), ONPG (+), ADH (-), LDC (-), ODC (-), citrate (-), sulfure d'hydrogène (-), uréase (-), tryptophane désaminase (+), indole (-), Voges-Proskauer (+), gélatinase (+), oxydase (-), glucose (+), mannose (+), inositol (-), sorbitol (-), rhamnose (+), sucrose (+), mélibiose (+), amidon (+), arabinose (+), réduction des nitrates (+) ;
- un profil électrophorétique de macrofragments de digestion de l'ADN génomique via les enzymes endonucléases de restriction *Xba* I et *Swa* I, constitué de 12 bandes de 306, 268, 255, 246, 233, 178, 153, 146, 141, 120, 87 et 77 kbases avec *Xba* I, et 13 bandes de 312, 304, 290, 235, 188, 168, 162, 150, 141, 122, 121, 111 et 86 kbases avec *Swa* I ;
- elle possède un marqueur de résistance à la rifampicine.

3. Souche bactérienne biofongicide selon la revendication 1, **caractérisée par le fait qu'**elle possède une dose moyenne efficace de 10⁶ à 10⁷ ufc/mL contre *Penicillium expansum.*

4. Souche bactérienne biofongicide selon la revendication 3, **caractérisée par le fait qu'**elle est également efficace lorsqu'elle est appliquée simultanément et jusqu'à 6 heures après ledit pathogène *Penicillium expansum.*

5. Souche bactérienne biofongicide selon la revendication 1, **caractérisée par le fait qu'**elle est compatible avec des fongicides synthétiques choisis parmi le folpet, l'imazalil, le myclobutanil, le thiabendazole, le thirame, le bénomyle, le captane, la procymidone, le tébuconazole, la diphénylamine et le méthyl-thiophanate.

6. Souche bactérienne biofongicide selon la revendication 1, **caractérisée par le fait qu'**elle est compatible avec l'application de sels aux concentrations utilisées normalement après la récolte.

7. Souche bactérienne biofongicide selon la revendication 6, **caractérisée par le fait que** lesdits sels sont le chlorure de calcium et le bicarbonate de sodium.

8. Souche bactérienne biofongicide selon la revendication 1, **caractérisée par le fait qu'**elle ne produit pas d'antibiose *"in vitro"* lorsqu'elle est testée dans des cultures doubles contre *Erwinia amylovora, Pseudomonas syringae, Penicillium expansum* ou *Stemphylium vesicarium,* dans des milieux de culture différents tels que des milieux glucose-asparagine, King B, Luria-Bertani ou pomme-de-terre-dextrose, qu'ils soient ou non supplémentés de Fe.

9. Souche bactérienne biofongicide selon la revendication 1, **caractérisée par le fait qu'**elle n'est ni pathogène ni toxique pour les plantes, les animaux ou les humains.

10. Souche bactérienne biofongicide selon la revendication 1, **caractérisée par le fait qu'**elle a un grand taux de survie cellulaire sur les surfaces de fruits et de légumes traités avec celle-ci.

11. Souche bactérienne biofongicide selon la revendication 10, **caractérisée par le fait que** ledit taux de survie est de l'ordre de 10⁴ à 10⁵ ufc/cm² inoculées à des poires et à des pommes à des doses comprises entre 10⁶ à 10⁸ ufc/cm², et dont la population augmente jusqu'à 10⁷ à 10⁸ ufc/cm² après 12 à 24 heures à 15°C et après 5 à 10 jours à 4°C lorsque des plaies sont produites sur la surface de la peau de ces fruits.

12. Procédure d'obtention de la nouvelle souche bactérienne CECT 5392 définie selon les revendications 1 à 11 ci-dessus, **caractérisée en ce qu'**elle consiste en l'homogénéisation du fruit du poirier *Pyrus communis* de la variété Doyenne du Comice dans de l'eau tamponnée peptonée ; en l'obtention d'un extrait contenant les microorganismes de l'homogénat correspondant suivie par la concentration de celui-ci par centrifugation ; en l'application dudit extrait concentré préalablement dilué dans un tampon sur des disques de pommes qui seront ensuite inoculés avec P. expansum, et finalement, en l'isolement et en la préparation d'une culture pure à partir de ces extraits qui s'avèrent eux-mêmes efficaces pour inhiber l'infection.

13. Procédure selon la revendication 12, **caractérisée en ce que** la souche ainsi obtenue peut être maintenue et conservée à court terme par les moyens d'un réensemencement dans de la gélose LB, à 2 à 4°C.

14. Procédure selon la revendication 12, **caractérisée en ce que** la souche ainsi obtenue peut être maintenue et conservée à moyen terme, c'est-à-dire, de 1 à 3 ans, par les moyens de surgélation à -80°C de solutions de la souche dans du glycérol à 20 % (w/v).

15. Procédure selon la revendication 12, **caractérisée en ce que** la souche ainsi obtenue peut être maintenue et conservée à long terme (plusieurs années) par les moyens de la lyophilisation de solutions de la souche dans du lait écrémé.

16. Procédure selon la revendication 12, **caractérisée en ce que** la souche ainsi obtenue peut être obtenue à grande échelle par les moyens de la production de masse dans un fermenteur en utilisant les opérations suivantes:
a) une culture pré-inoculum est préparée dans un milieu MSD jusqu'à atteindre la phase stationnaire, qui est alors utilisée pour inoculer le bioréacteur fonctionnant à 25 à 28°C, pH 7,0, 250 à 400 tr/min et un courant d'air de 30 à 60 lpm. Après 4 à 5 heures, une solution de 200 à 400 g de glucose est ajoutée et la fermentation se poursuit pendant jusqu'à 8 à 12 heures ;
b) la centrifugation continue à 10 000 tr/minute et 20 à 30 1/h et les cellules sont collectées de manière aseptique, en maintenant les conditions de fonctionnement du bioréacteur pendant 2 à 3 heures ;
c) la biomasse du réacteur est concentrée pendant 2 à 3 heures et remise en suspension dans un tampon phosphate, pour obtenir la souche à grande échelle.

17. Procédure selon l'une quelconque des revendications 12 à 16, **caractérisée par le fait que** la souche CECT 5392 peut être améliorée en termes de son efficacité fongicide par les moyens d'une étape supplémentaire d'osmoadaptation qui est réalisée par traitement de celle-ci avec de la bétaïne et du chlorure de sodium.

18. Utilisation de la souche CECT 5392 définie par l'une quelconque des revendications 1 à 11 ci-dessus, dans la fabrication de préparations biofongicides pour une utilisation commerciale qui comprennent ladite souche ou un extrait ou un dérivé actif de celle-ci, ou un mutant de celle-ci, qui a essentiellement les mêmes marqueurs moléculaires ou phénotypiques spécifiques.

19. Utilisation selon la revendication 18, **caractérisée en ce que** lesdites préparations biofongicides incorporent, outre la souche, un fongicide chimique conventionnel.

20. Utilisation selon la revendication 19, **caractérisée en ce que** ledit fongicide chimique conventionnel est choisi dans un groupe constitué par le folpet, l'imazalil, le myclobutanil, le thiabendazole, le thirame, le bénomyle, le captane, la procymidone, le tébuconazole, la diphénylamine et le méthyl-thiophanate.

21. Utilisation selon l'une quelconque des revendications 18 à 20, **caractérisée en ce que** lesdites préparations biofongicides sont mélangées, formulées ou diluées avec un composé acceptable sur le plan agricole de manière à améliorer leur adhérence, leur mouillabilité, à fournir des éléments nutritifs et/ou à améliorer les chances de survie de la souche.

22. Utilisation selon l'une quelconque des revendications 18 à 21, **caractérisée en ce qu'**elle est destinée à lutter contre les infections fongiques produites par des champignons phytopathogènes.

23. Utilisation selon la revendication 22, **caractérisée en ce que** ledit champignon phytopathogène appartient au genre *Penicillium.*

24. Utilisation selon l'une quelconque des revendications 18 à 23, **caractérisée en ce qu'**elle est destinée à la protection d'un fruit contre les infections fongiques.

25. Utilisation selon la revendication 24, **caractérisée en ce que** ledit fruit est protégé à l'état pré-récolte, par l'application desdites préparations biofongicides aux arbres dans les champs où ils sont cultivés.

26. Utilisation selon la revendication 24, **caractérisée en ce que** ledit fruit est protégé à l'état post-récolte durant sa période de stockage et de conservation.

27. Utilisation selon les revendications 24 à 26, **caractérisée en ce que** ledit fruit est un fruit à pépins.

28. Utilisation selon l'une quelconque des revendications 18 à 27, **caractérisée en ce que** ladite souche est appliquée aux installations, aux équipements ou aux conteneurs destinés à être en contact avec ledit fruit.
